# EUROPEAN PATENT SPECIFICATION

(11) **EP 1 653 779 B1**
(45) Date of publication and mention of the grant of the patent: **24.06.2009**
(21) Application number: 05104337.0
(22) Date of filing: 23.05.2005
(51) Int. Cl.: H05B 3/14, H01C 7/02, A01M 1/20, A01M 13/00, A61L 9/03

(54) **PTC electric heating device for vaporisers of fragances or insecticides in the form of solid mats**
Elektrische PTC-Heizvorrichtung für Verdampfer von Duftstoffen oder Insektiziden in der Form von Feststoffmatten
Dispositif de chauffage électrique à coefficient de température positif pour vaporisateurs parfums ou insecticides sous forme de natte de matière solide

(30) Priority: 28.10.2004 IT MI20042055
(43) Date of publication of application: 03.05.2006
(73) Proprietor: ZOBELE HOLDING S.P.A., 38100 Trento (IT)
(72) Inventor: Zobele, Franco, Trento 38100 (IT)
(74) Representative: Faggioni, Marco

(56) References cited:
- EP-A- 0 716 807
- EP-A- 0 965 267
- EP-A- 1 029 451
- WO-A-97/02054
- DE-A1- 19 806 269
- US-A1- 2002 166 853

## Description

The present invention relates to a PTC heating device for vaporisers of fragrances or insecticides in the form of solid mats.

In the field of electric fragrance or insecticide vaporisers for domestic use, the use of so-called PTCs (Positive Temperature Coefficient devices) is by now largely widespread, which PTCs have in fact a number of advantages, in terms of thermo-electric performance, over the electric resistors previously used.

However, since the cost of these devices is higher than that of an electric resistor of equal power, field research is now particularly addressing the issue of cost reduction of vaporisers, both in terms of costs of material, and especially of assembly costs. Attempts have therefore been made first of all to reduce the widespread use of labour traditionally employed for the assembling of these devices, re-designing vaporisers and the corresponding components so as to allow at least part of the assembling to be carried out automatically. On the other hand, an attempt has also been made to reduce the number of components, decreasing accordingly the number of operations required for the assembly and consequently the cost of the product.

Research has further addressed the standardisation of vaporisers and in particular of inner components thereof which are capable of keeping their structure unchanged also in vaporisers with a varying outer appearance. Over time the field industry has in fact had to face two opposite problems: on the one hand the above-mentioned need to curb manufacturing costs which pushed towards full product standardisation; on the other hand, market requirements demanding vaporisers with a frequently-changing appearance to suit the public's constantly evolving taste.

These opposite requirements were hence satisfyingly met moving from an "individual" design approach, wherein the entire vaporiser used to be designed in view of a specific support onto which the individual inner components used to be mounted directly, to a "modular" design approach, wherein the different technical inner components - and particularly the vaporiser heating device among these - are designed independently from the specific vaporiser and highly standardised, so as to make production thereof easily automatable and fully independent from that of the outer support wherein they are subsequently introduced during a final assembly operation. It is also to be appreciated that this final assembly operation is itself easily automatable, despite the non-standardisation of the different types of outer supports, since in this case the mounting process includes relatively large-sized components which may therefore be more easily oriented and handled, with no need of particularly sophisticated automatic machines.

An example of the known prior art for the "individual" design is illustrated for example in DE-A-91 04 709, wherein all the components are in fact assembled directly on the outer support.

The present invention addresses instead the field of "modular" vaporiser design and is specifically directed at the production of the essential inner component of said vaporisers, that is, of a PTC electric heating device which is self-supporting and self-contained, complete with a PTC element and with the relative electric connectors and protections and further equipped with a heating surface whereon a mat imbued with a fragrance or insecticide can be placed directly in contact, once said device has been introduced in a suitable outer support.

Heating devices falling into the category defined above are already known in the art. Such devices initially consisted of a container entirely made of ceramics wherein the PTC element and the relative metallic contacts were housed. Such containers were later virtually abandoned, substantially due to the difficulty of manufacturing moulded ceramics pieces with a sufficiently small and constant thickness so as to guarantee good heat flow and temperature evenness in the mat imbued with a vaporisable substance.

Containers have later been suggested wherein the container consists of a ceramics or plastic body, while the part of the container in contact with the mat imbued with the vaporisable substance consists of a metal sheet, such as those described for example in GB-A-2 181 629 and WO 97/45008. In this type of heating devices, improved evenness of heat distribution is obtained, thanks to the presence of the metal sheet, but there are still problems as far as electric protections and locking of the different device components are concerned.

With a view to electric safety, the presence of the outer metal sheet is currently not an appreciated solution, due to the fact that it represents a potential danger for electric shock to the user - especially to children, who may accidentally introduce metal objects in the vaporiser housing intended to house the mat - should the film of insulating material lying between the electric contact and the outer metal sheet have defects in the material or in the assembly thereof.

Further, concerning the mutual locking of the various components of a heating device, all the different solutions provides by the known art require the production of specially designed pieces, and hence higher manufacturing costs. In the case of the British patent, in fact, the metal sheet has side walls apt to determine a housing seat for the ceramic support and also a series of flaps departing from said walls to be clenched underneath the ceramic body and thereby performing locking thereof. In the PCT publication the specially designed piece is instead the plastic support, which in fact has a plurality of elastically flexible teeth, with which the metal sheet engages, which in this case is flat. WO 97/02054 discloses a PTC heatin device which features are the preamble of the claim 1 of the present invention.

It is hence an object of the present invention to provide a device of the general type illustrated above which eliminates the drawbacks mentioned above, by offering a high level of electric safety and a simplified locking system of the various components thereof.

Such object is achieved according to the present invention by an electric heating device for vaporisers of fragrances or insecticides having the features defined in the main claim herewith enclosed.

Further features and details of the heating device of the invention will in any case be more evident from the following detailed description of a preferred embodiment thereof, shown in the accompanying drawings, wherein:

fig. 1 is an exploded view of a heating device according to the invention, seen from the electric contacts side;

fig. 2 is an exploded view of the heating device of fig. 1, seen from the side of the resting plane of the mat imbued with active substance; and

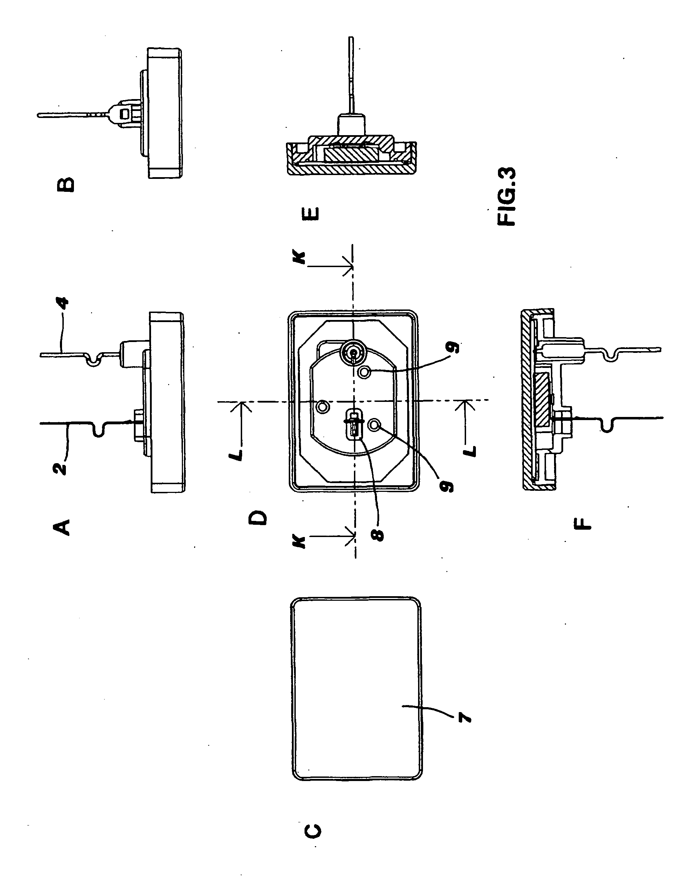

fig. 3 is a drawing showing, in the various views specified below, the assembled device of the invention:
A a front elevation view;
B a side elevation view;
C a bottom plan view;
D a top plan view;
E a cross-section view according to line L-L of fig. 3D;
F a cross-section view according to line K-K of fig. 3D.

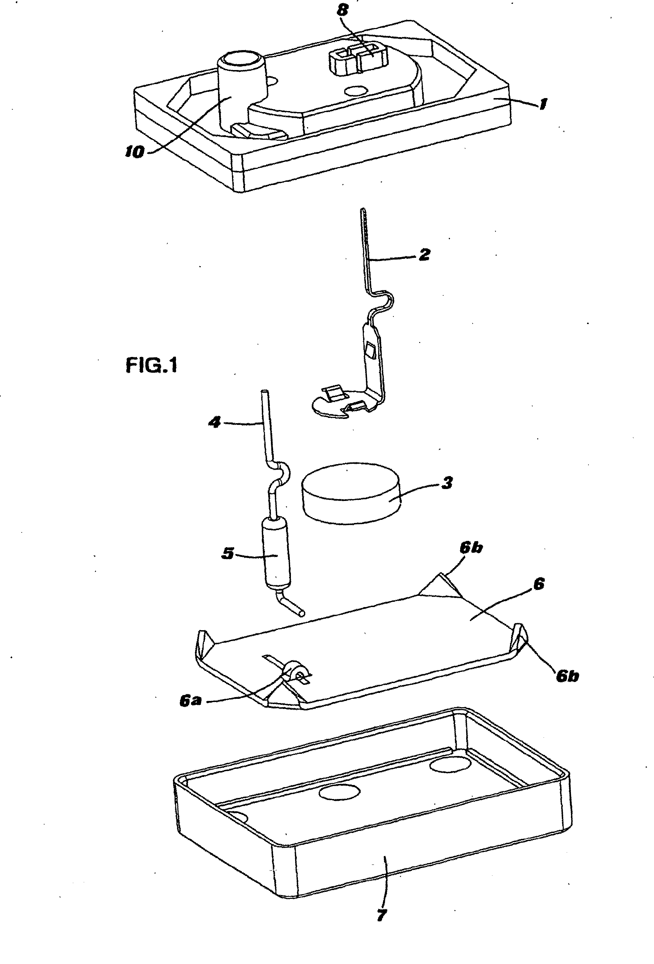

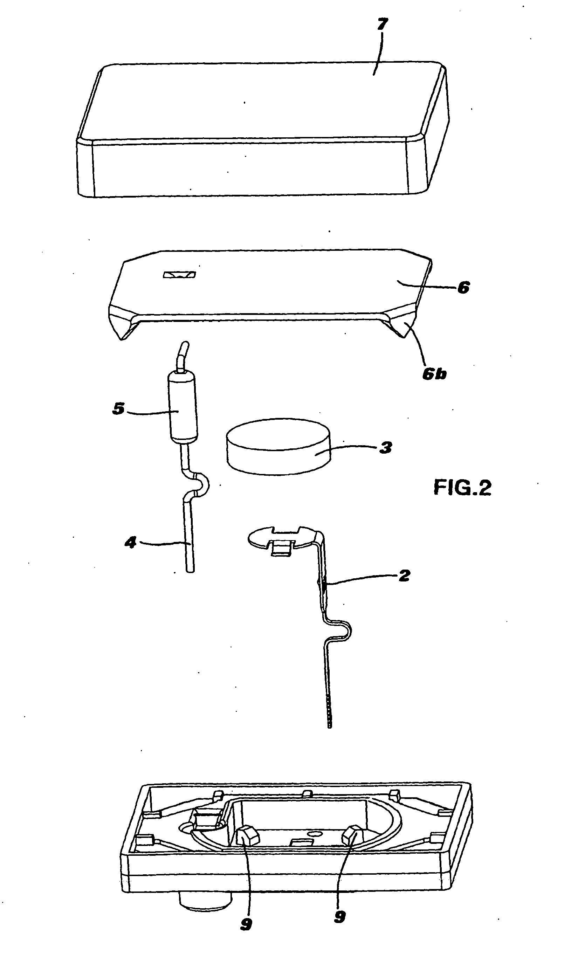

The heating device according to the invention has an extremely simple and highly functional structure. As is clearly visible from figs. 1 and 2, it consists of a rectangular plastic support 1, intended to house within it a first electric contact 2, a PTC heating element 3, a second electric contact 4, integrally equipped with a fuse 5, a metal sheet 6, and finally a cover 7 of support 1, itself made of plastic.

During device assembly, first electric contact 2 is introduced in a suitable recess of support 1, the flat circular part of contact 2 arranging itself in a housing formed in said recess and marked out by three teeth 9. The elongated portion of contact 2 is instead introduced in an eyelet 8 provided on the floor of support 1 and projects therefrom for connection with an electric plug, in a manner well-known per se. The seat for housing the flat portion of contact 2, formed by the three teeth 9, is also intended to house PTC 3 which is in fact placed on top of such contact 2 whereon a pair of elastically deformable flaps are further provided for improved electric contact and in a manner known per se.

After this first assembly step, the second electric contact 4 is secured to metal sheet 6 by introducing one end thereof below U-bolt 6a provided on said sheet, which U-bolt is then clinched to perform mechanical fixing and electric connection between contact 4 and sheet 6. Thereby sheet 6 hence simultaneously acts both as an electric contact for the PCT and as a diffuser of the heat generated by the same, which functions were instead performed by two different elements in known devices. Sheet 6 is then steadily positioned in support 1, thanks to its four bent-down corners 6b which are housed in respective seats formed in support 1, whereas the elongated portion of contact 4, in a fully similar way to the corresponding portion of contact 2, enters a cylindrical slit 10 of support 1 projecting therefrom at the opposite side. Once that has been accomplished, the fuse 5 connected with contact 4 is completely encircled by a cylindrical safety wall, formed integrally with support 1 in correspondence of slit 10, such wall thereby preventing the outer shell of the vaporiser from being punctured by the fragments of fuse 5, in the event of the same blowing due to a short-circuit.

Assembly of the heating device is completed by cover 7 which entirely covers metal sheet 6 and, with its side walls, also fully covers the side walls of support 1, whereon it is welded by ultrasonic thermo-welding, thereby accomplishing quick device locking, which is nevertheless extremely stable and may not be further opened. The flat outer surface of cover 7 is hence suitable to be used as a resting surface for mats imbued with fragrances or insecticides, thanks to the heat being distributed therein in a particularly even and regular manner, due to underlying metal sheet 6 and also to the small and utterly constant thickness of cover 7.

The device thus assembled is ready for insertion in a corresponding vaporiser, after a keeper (not shown) has been fitted to the free end of contacts 2 and 4, provided with plugs of the desired type for insertion in an electric socket. Once the keeper has been clamped on the free ends of contacts 2 and 4, it is introduced in a suitable seat of the vaporiser and pulled in position against the action of snap-fitting elastic teeth which determine permanent fixing thereof. However, during this operation it may sometimes happen that the tensile stress imparted to the plugs is unduly transferred also onto electric contacts 2 and 4; hence in order to avoid any possible damage to such contacts, or to the connections thereof, the elongated portion of each of contacts 2 and 4 has a typical U-shaped loop, which is clearly visible in the drawings and which is capable of imparting to the contacts a sufficient degree of longitudinal elasticity to absorb any stress which may discharge thereon during assembly operations.

From the preceding description it should be clear how the present invention has fully achieved the desired objects by providing a heating device with a very simple and linear construction which can hence be easily manufactured in an automated way. Said device has a high degree of electric safety, thanks to the fact that it has no outside metallic surfaces, that a fuse-based short-circuit protection system is further provided, as well as a further protection from the ejection of any splinters in case of blowing of said fuse, and finally that the locking system adopted, in addition to being very simple, is also effective against any voluntary or accidental re-opening attempt of the device.

## Claims

1. A PTC electric heating device for vaporisers of fragrances or insecticides imbued on solid mats, of the type suitable to be introduced in the body of a vaporiser as an electrically-insulated self-contained unit and comprising a plastic support (1) for housing the PTC element (3), respective electric contacts (2, 4-6), and a flat plastic cover (7) of said support, **characterised in that** said cover is suitable to act, with its outer side, as a resting surface of said mats and further comprising a metal sheet (6) for heat diffusion placed in contact with said cover (7) and said PTC element (3), said metal sheet (6) having an extension substantially corresponding to that of said cover (7) and representing one of said electric contacts (4-6), said electric contacts (2, 4-6) comprising elongated portions which protrude from suitable apertures (8, 10) of said support (1).

2. The device as in claim 1) ; wherein said cover (7) is fixed to the corresponding support (1) by ultrasonic thermal welding.

3. The device as in claim 1), wherein said cover (7) comprises side walls apt to overlap corresponding walls of the support (1), ultrasonic thermo-welding occurring in correspondence of such overlapping walls.

4. The device as in claim 1), therein one of said electric contacts (4-6) comprises a fuse (5) and said fuse is housed in a cylindrical safety housing (10) formed in said support in correspondence of one of said apertures for the passage of the elongated portions of said contacts.

5. The device as in claim 1), therein the elongated portions of said contacts have corresponding U-shaped loops to impart longitudinal elasticity to the contacts during the assembly operation of a plug on said contacts.

## Patentansprüche

1. Elektrische PTC-Heizvorrichtung für Verdampfer von Duftstoffen oder Insektiziden, die auf Feststoffmatten getränkt sind, der Art, die dazu geeignet ist, in den Körper eines Verdampfers als eine elektrisch isolierte abgeschlossene Einheit eingeführt zu werden, und die einen Kunststoffträger (1) zur Aufnahme des PTC-Elements (3), entsprechende elektrische Kontakte (2, 4-6) und eine flache Kunststoffabdeckung (7) des Trägers umfasst, **dadurch gekennzeichnet, dass** die Abdeckung dazu geeignet ist, um mit ihrer äußeren Seite als eine Stützfläche besagter Matten zu wirken und ferner ein Metallblech (6) zur Wärmediffüsion umfasst, das in Kontakt mit der Abdeckung (7) und dem PTC-Element (3) angeordnet ist, wobei das Metallblech (6) eine Erstreckung aufweist, die im wesentlichen derjenigen besagter Abdeckung (7) entspricht und einen besagter elektrischer Kontakte (4, 6) darstellt, wobei besagte elektrische Kontakte (2, 4-6) längliche Abschnitte umfassen, die aus geeigneten Öffnungen (8, 10) des Träges (1) hervorstehen.

2. Vorrichtung nach Anspruch 1, wobei die Abdeckung (7) an dem entsprechenden Träger (1) durch thermisches Ultraschallthermoverschweißen befestigt ist.

3. Vorrichtung nach Anspruch 1, wobei die Abdeckung (7) Seitenwände umfasst, die passend sind, um mit entsprechenden Wänden des Trägers (1) zu überlappen, wobei ein Ultraschallthermoverschweißen bis Übereinstimmung mit solchen überlappenden Wänden auftritt.

4. Vorrichtung nach Anspruch 1, wobei einer besagter elektrischer Kontakte (4-6) eine Sicherung (5) umfasst und besagte Sicherung in einem zylindrischen Sicherheitsgehäuse (10) aufgenommen ist, das in besagtem Träger bis Übereinstimmung mit einer besagter Öffnungen für den Durchgang der länglichen Abschnitte besagter Kontakte gebildet ist.

5. Vorrichtung nach Anspruch 1, wobei die länglichen Abschnitte besagter Kontakte entsprechende U-förmige Schlaufen aufweisen, um den Kontakten während des Zusammenbaus eines Steckers an den Kontakten Längselastizität zu vermitteln.

## Revendications

1. Dispositif de chauffage électrique à coefficient positif de température PTC pour des vaporiseurs de parfums ou d'insecticides imbibés sur des mats solides, du type apte à être introduit dans le corps d'un vaporiseur sous la forme d'une unité autonome isolée électriquement et comportant un support (1) en matière plastique destiné à loger l'élément PTC (3), des contacts électriques respectifs (2, 4-6) et un capot plat (7) en matière plastique dudit support, **caractérisé en ce que** ledit capot est apte à agir, par son côté extérieur, en tant que surface sur laquelle repose lesdits mats et comportant en outre une feuille métallique (6) pour la diffusion de la chaleur placée en contact avec ledit capot (7) et ledit élément PTC (3), ladite feuille métallique (6) ayant une étendue correspondant sensiblement à celle dudit capot (7) et représentant l'un desdits contacts électriques (4-6), lesdits contacts électriques (2, 4-6) comportant des parties allongées qui font saillie d'ouvertures appropriées (8, 10) dudit support (1).

2. Dispositif selon la revendication 1, dans lequel ledit capot (7) est fixé au support correspondant (1) par un soudage thermique par ultrasons.

3. Dispositif selon la revendication 1, dans lequel ledit capot (7) comporte des parois latérales aptes à chevaucher des parois correspondantes du support (1), un soudage thermique par ultrasons ayant lieu au niveau de ces parois qui se chevauchent.

4. Dispositif selon la revendication 1, dans lequel l'un desdits contacts électriques (4-6) comporte un fusible (5) et ledit fusible est logé dans un boîtier cylindrique (10) de sureté formé dans ledit support au niveau de l'une desdites ouvertures pour le passage des parties allongées desdits contacts.

5. Dispositif selon la revendication 1, dans lequel les parties allongées desdits contacts ont des boucles en forme de U correspondantes pour conférer une élasticité longitudinale aux contacts pendant l'opération d'assemblage d'une fiche sur lesdits contacts.
